(19) Europäisches Patentamt
European Patent Office
Office européen des brevets

(11) Publication number: **0 265 166 B1**

(12) **EUROPEAN PATENT SPECIFICATION**

(45) Date of publication of patent specification: **04.09.91**

(51) Int. Cl.[5]: **C12P 37/00**, //C07F7/18, C07D499/00

(21) Application number: **87309078.1**

(22) Date of filing: **14.10.87**

# (54) Process for preparing penems.

(30) Priority: **15.10.86 GB 8624686**

(43) Date of publication of application:
**27.04.88 Bulletin 88/17**

(45) Publication of the grant of the patent:
**04.09.91 Bulletin 91/36**

(84) Designated Contracting States:
**AT BE CH DE ES FR GB GR IT LI LU NL SE**

(56) References cited:
**EP-A- 0 044 736**
**US-A- 4 053 360**

(73) Proprietor: **FARMITALIA CARLO ERBA S.r.L.**
**Via Carlo Imbonati 24**
**I-20159 Milano(IT)**

(72) Inventor: **Altamura, Maria**
**Viale Volta 85/G**
**I-28100 Novara(IT)**
Inventor: **Cesti, Pietro**
**Via Torino, 51**
**I-28069 S. Martino Di Trecate (Novara)(IT)**
Inventor: **Francalanci, Franco**
**Via Galileo Ferraris, 7**
**I-28100 Novara(IT)**
Inventor: **Marchi, Marcello**
**Viale Allegra, 11**
**I-28100 Novara(IT)**
Inventor: **Foa', Marco**
**Via Del Sabbione, 19**
**I-28100 Novara(IT)**
Inventor: **Cambiaghi, Stefano**
**Via Eredi Farina, 16**
**I-27100 Pavia(IT)**
Inventor: **Dallatomasina, Franco**
**Via S. Felice Torre, 4**
**I-20090 Segrate (Milan)(IT)**

(74) Representative: **Woods, Geoffrey Corlett et al**
**J.A. KEMP & CO. 14 South Square Gray's Inn**
**London WC1R 5EU(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel process for preparing 2-hydroxymethyl penems useful in the synthesis of penems having antibacterial activity.

More particularly the invention relates to a process for the preparation of compounds of the formula I :

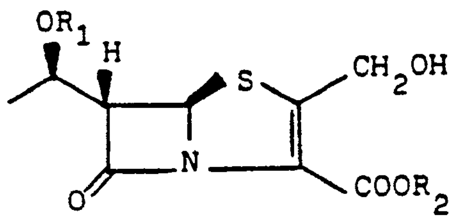

(I)

wherein $R_1$ represents a hydroxy protecting group and $R_2$ represents a carboxy protecting group, which process comprises hydrolysing a compound of the formula II :

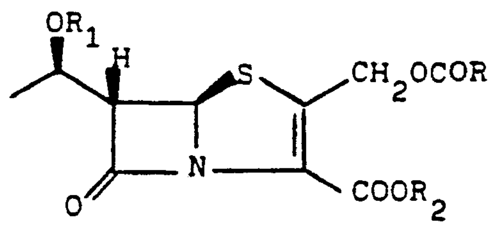

(II)

wherein $R_1$ and $R_2$ are as defined above and R represents an alkyl, alkenyl or phenylalkyl group having from 1 to 18 carbon atoms, by means of an enzyme capable of selectively hydrolysing the ester group of the 2-substituent thereof.

The hydroxy protecting groups which $R_1$ may represent include p-nitrobenzyloxycarbonyl, 2,2,2,-trichloro-ethoxy carbonyl, trimethylsilyl, benzyl, p-bromophenacyl, triphenylmethyl and pyranyl groups.

Preferred protecting groups which $R_1$ may represent are p-nitrobenzyloxycarbonyl, trimethylsilyl and pyranyl groups.

The carboxy protecting groups which $R_2$ may represent include

a) alkyl groups having from 1 to 6 carbon atoms,
b) haloalkyl groups having from 1 to 6 carbon atoms,
c) alkenyl groups having from 2 to 4 carbon atoms,
d) optionally substituted aryl groups,
e) optionally substituted aralkyl groups, the alkyl part whereof has from 1 to 6 carbon atoms and
f) aryloxyalkyl groups.

Examples of these are :

a) methyl, ethyl and t-butyl groups,
b) the 2,2,2-trichloroethyl group,
c) the allyl group,
d) phenyl and p-nitrophenyl groups,
e) benzyl, p-nitrobenzyl, p-methoxybenzyl, diphenylmethyl and di-(o-nitrophenyl)-methyl group and
f) the phenoxymethyl group.

Other groups which should be mentioned as representative carboxy protecting groups are acetonyl and trimethylsilyl groups. Also included are residues known to be hydrolyzed in vivo and to have favourable pharmacokinetic properties, such as acetoxymethyl, pivaloyloxymethyl and phthalidyl groups. The preferred carboxy protecting groups are allyl, benzyl and p-nitrobenzyl groups.

Preferred alkyl group which R may represent include methyl, ethyl, propyl, butyl, pentyl,hexyl. Preferred alkenyl groups are allyl, propenyl, butenyl. Preferred phenylalkyl groups include benzyl or phenethyl.

As stated above, compounds of formula I may be converted into known antibacterial agents, as in detail explained and claimed in our published U.K.Patent Application GB 2111496-A and GB 2118181-A. These known antibacterial agents, named penems, are described for example in British Patent Specifications 2043639-B, 2097786 B and in the published European Application 0167100-A.

As described in the above cited prior art, the compounds of the formula I are prepared by chemical selective hydrolysis of compounds of formula III :

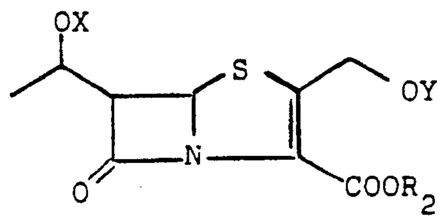

III

wherein $R_2$ is as defined above, and X and Y are two different silyl derivatives such as t-butyldimethyl silyl and t-butyldiphenylsilyl groups respectively, by means of tetraalkylammonium floride.

The synthesis of compounds III and said selective removal of protecting group Y require expensive reagents and long reaction times which are not suitable for industrial large scale preparation of penems.

The present invention provides a simple process for the preparation of compounds of the formula I by selective and inexpensive enzymatic hydrolysis of the compounds of the formula II as defined above.

The process of the invention, using enzymatic hydrolysis, allows the final product to be obtained under very mild conditions in very high yields and without undesired by-products.

The configuration of the compounds of the formulae II and III is [5R,6S,(1R)], in order to obtain the preferred final [5R,6S,(1R)] stereochemistry of the penum nucleus. The starting materials of the formula II are known compounds or may be prepared according to known procedures, for example as described in the published U.K. Patent Application GB 2144743-A.

Hydrolytic enzymes suitable for the present process are, for example, lipases or proteases which selectively hydrolyze the carboxylic ester of the 2-hydroxymethyl residue ($-CH_2OCOR$) of compound of the formula (II) without affecting other functional groups which may be present. The hydrolytic process can be carried out either by using directly free or immobilized microbial cells which secrete a suitable enzyme or by isolating the specific enzymes which can be used in the free form, immobilized according to known techniques to resins, glass, cellulose or similar substances by ionic or covalent bonds, or grafted to fibres permeable to the substrate, or insolubilized by cross-linkage. Immobilization or insolubilization is advantageous as the same enzyme can be used for many production cycles. Moreover when an immobilized enzyme is used the recovery of the reaction product is more easy. In fact, being the reaction product adsorbed on the resin at the end of the reaction it is easily recovered in pure form by simply washing the resin with a suitable solvent.

The use of the enzymes isolated and purified to the desired degree is preferred rather than the raw cellular extract since the extraction or purification process normally allows a reduction or elimination of the presence of contaminating enzymes which could lower the yields by formation of undesired by-products.

Also enzymatic preparation obtained by extraction of animal organs, such as porcine pancreas, are able to cause hydrolysis of the ester bond between the carbonyl group of an organic acid and the hydroxymethyl group in position 2 of the penem nucleus.

Commercially available hydrolytic enzymes can be used in the hydrolytic process, for example.

| Enzyme | Origin | Seller |
|---|---|---|
| Pancreatin | Porcine pancreas | UNIBIOS - Trecate (Italy) |
| Steapsin | Porcine pancreas | SIGMA Chem. Co. St.Louis (U.S.A.) |
| Lipase | Candida Cylindracea | " |
| Lipase | Wheat germ | " |
| Lipase SP 225 | | NOVO Industri (Denmark) |
| Lipase | Rhizopus Delamar | SIGMA Chem.Co. St.Louis (U.S.A.) |
| Lipase | Chromobacterium Viscosum | TOYO JOZO (Japan) |
| Lipoprotein Lipase | Pseudomonas Sp. | TOYOBO (Japan) |
| Protease | Streptomyces Caespitosus | SIGMA Chem.Co. |
| Protease | Rhizopus Sp. | SIGMA Chem.Co. |

The enzymes may be added to an aqueous suspension of from 1 to 100 g/l of the ester of the formula II optionally containing small amounts of hydrocarbons, and suitably mildly buffered at different pH according to the enzyme used, that is in a range from 5 to 9, preferably from 6 to 8.

The reaction may be carried out at a temperature of from 10° C to 50° C, preferably from 20° C to 40° C, for 0.5 to 48 hours, operating in batch or column, according to the quantity of the enzyme present in the reaction mixture, and to the ratio between the quantity of the enzyme in solution or in the immobilized form, and the quantity of substrate present in the reaction mixture.

The pH of the reaction mixture is kept constant at the desired value by adding a solution of an alkali hydroxide.

The yields of the reaction carried out under optimal conditions reach values higher than 90%.

At the end of the reaction, the reaction product is recovered by conventional methods.

Hereunder, the present invention will be more fully described by means of the following examples, which, however, should not be construed to be limitative.

Preparation A)

Allyl (5R,6S)-2-butyryloxymethyl-6-[1(R)-trimethylsilyloxyethyl]-penem-3-carboxylate.

4.27 g of (3S,4R)-4-butyryloxyacetylthio-3-(1(R)-trimethylsilyloxyethyl)-2-azetidinone were dissolved in 40 ml dry toluene.

700 mg calcium carbonate and 2.2 g allyl oxy oxalylchloride were added to the solution under nitrogen atmosphere, at a temperature of 10° C.

2.1 ml triethylamine were added dropwise to the mixture, at the same temperature, over a 30 min. period. At the end of the addition, the mixture was stirred 10′ at 10° C.

Calcium carbonate was filtered off and the solution washed with water, 5% $NaHCO_3$, water. After drying over $Na_2SO_4$ the solution was concentrated to 20 ml, added with 4 7 ml triethylphosphite and refluxed 6 h.

The reaction mixture was cooled at 20° C washed with water (3 x 10 ml) and dried over $Na_2SO_4$.

Evaporation of the solvent gave a crude oil, which was chromatographed on silica gel (ethyl

ether/hexane 3 : 7 v/v) to afford 2 6 g of pure allyl (5R,6S)-2-butyryloxymethyl-6-[1(R)-trimethylsilyloxyethyl]-penem-3-carboxylate (50%).

NMR (300 NHz, $CDCl_3$) - δ (ppm)

0.13 [9H, s, $Si(CH_3)_3$]
0.95 (3H, t, $OCOCH_2CH_2$ $CH_3$)
1.25 (3H, d, $CH_3CH$)
1.7 (2H, m, $OCOCH_2$ $CH_2CH_3$)
2.3 (2H, t, OCO $CH_2CH_2CH_3$)
3.7 (1H, dd, H-6)
4.2 (1H, m, H-8)
4.7 (2H, m, $CH_2$-CH = $CH_2$)
5.2-5.5 (2H, m, CH = $CH_2$)
5.05-5.55 (2H, m, $CH_2OCO$)
5.55 (1H, d, H-5) - 5.9-6.0 (1H, m, CH = $CH_2$).

Preparation B)

Allyl (5R,6S)-2-acetyloxymethyl-6-[1(R)-trimethylsilyloxyethyl]-penem-3-carboxylate.

The preparation was carried out as described in A), starting from (3S,4R)-4-acetoxyacetylthio-3-[1-(R)-trimethylsilyloxyethyl]-2-azetidinone.

Allyl (5R,6S)-2-acetyloxymethyl-6-[1(R)-trimethylsilyloxyethyl]-penem-3-carboxylate was obtained as a pure product in an overall yield of 48%.

NMR (300 NHz, $CDCl_3$)- δ (ppm) :

0.15 (9H, s, $Si(CH_3)_3$)
1.28 (3H, d, $CH_3CH$)
2.1 (3H, s, CO $CH_3$)
3.72 (1H, dd, J=2Hz, 6Hz, H-6)
4.21 (1H, m, H-8)
4.65-4.80 (2H, m, COO $CH_2$-CH=)
5.05-5.55 (2H, m, $CH_2OCO$)
5.25-5.5 (2H, m, CH = $CH_2$)
5.55 (1H, d, J=2Hz, H-5)
5.85-5.60 (1H, m, CH = $CH_2$)

Preparation C

Allyl (5R,6S)-2-butyryloxymethyl-6-[1(R)-tetranyaropyranyloxyethyl]-penem-3-carboxylate.

The prerparation was carried out as described in A), starting from (3S,4R)-4-butyryloxyacetylthio-3-[1-(R)-tetrahydropyranyloxyethyl]-2-aze idinone.

Allyl (5R,6S)-2-butyryloxymethyl-6-[1(R)-tetrahydropyranyloxyethyl]-penem-3-carboxylate was obtained as a pure product, in an overall yield of 45%.

NMR (300 MHz, $CDCl_3$) - δ (ppm) :

0.95 (3H, t, J=6.7 Hz,$COCH_2CH_2$ $CH_3$)
1.30-1.37 (3H, m, $CH_3$-CH)
1.42-1.90 (6H, m, $CH_2CH_2CH_2$ of tetrahydropyranyl group)
1.67 (2H, m,$COCH_2$ $CH_2CH_3$)
2.32 (2H, t,CO $CH_2CH_2CH_3$)
3.4-3.9 (2H, m, $OCH_2$ of tetrahydropyranyl group)
3.8 (1H, m, H-6)
4.05-4.2 (1H, m, H-8)
4.6-4.85 (3H : COO $CH_2$-CH= and CH of tetrahydropyranyl)
5.05-5.5 (2H, m, $CH_2OCO$)
5.2-5.42 (2H, m, CH= $CH_2$)
5.6 (1H, m, H-5)
5.85-6.0 (1H, m, CH = $CH_2$)

EXAMPLE 1

5 g of allyl (5R,6S)-2-butyryloxymethyl-6-[1-(R)-trimethylsilyloxyethyl]-penem-3-carboxylate, dissolved in 5ml n-hexane, were added to 300 ml of phosphate buffer 0.05N (pH = 7.5). The mixture was added with 25 mg of lipase from Chromobacterium Viscosum and stirred at 30° C for 4 hours.

The pH was kept at 7.50 by addition of 1N NaOH.

At the end of the reaction, the reaction mixture was extracted with $CH_2Cl_2$ (3 X 100 ml); the organic layer was dried over sodium sulfate and evaporated to give 3,9 g of pure allyl (5R,6S)-2-hydroxymethyl-6-[1(R)-trimethylsilyloxyethyl]-penem-3-carboxylate (93%).

$^1$H-NMR (300 MHz, $CDCl_3$) - $\delta$ (ppm) :
0.15 (9H, s, $Si(CH_3)_3$)
1.32 (3H, d, J = 6.5 Hz, $CH_3$-CH)
3.75 (1H, dd, J = 2Hz, 6.5 Hz; H-6)
3.85 (1H, br, OH)
4.25 (1H, m, H-8)
4.65 (2H, s, $CH_2OH$)
4.65-4.95 (2H, m, $CH_2$-CH =)
5.25-5.5 (2H, m, CH = $CH_2$)
5.60 (1H, d, J = 2Hz, H-5)
5.95-6.05 (1H, m, CH = $CH_2$)

EXAMPLE 2

The reaction was carried out as described in Example 1, except that the enzyme used was Lipoprotein Lipase (Toyobo) having an activity of 10 U/mg solid.

The mixture was stirred at 30° C for 2 hours and the product recovered as described in Example 1, obtaining a yield of 94%.

EXAMPLE 3

4.5 g Of allyl (5R,6S)-2-acetoxymethyl-6-[1(R)-trimethylsilyloxyethyl]-penem-3-carboxylate were added to 300 ml of phosphate buffer 0.05N (pH = 7.0).

The mixture was added with 25 mg of lipase from Chromobacterium viscosum and stirred at 30° C for 5 hours.

The pH was kept at 7.0 by addition of 1N NaOH.

At the end of the reaction, the reaction mixture was extracted with $CH_2Cl_2$ (3 x 100 ml). The extracts were dried over sodium sulfate and evaporated to give allyl (5R,6S)-2-hydroxymethyl-6-[1 (R)-trimethylsilyloxyethyl]-penem-3-carboxylate with a yield of 95%.

EXAMPLE 4

The reaction was carried out as described in Example 1, except that the enzyme used was Pancreatin-(Unibios; 3.5 g).

The mixture was stirred at 30° C for 20 h (pH = 7.5). The product was recovered as described in Example 1, with a yield of 91%.

EXAMPLE 5

The reaction was carried out as described in Example 1, except that the enzyme used was Pancreatin (3.5 g). The mixture was stirred at 30° for 22 h (pH = 8.0). At the end of the reaction, the mixture was extracted with $CH_2Cl_2$ (3 x 100 ml).

The extracts were concentrated under a reduced pressure and chromatographed on a column of silica gel, eluting with a mixed solvent of hexane-ethyl ether (20 : 80 v/v).

Evaporation of the solvent afforded 1.67 g (40%) of allyl (5R,6S)-2-hydroxymethyl-6-[1(R)-trimethylsilyloxyethyl-penem-3-carboxylate.

EXAMPLE 6

The reaction was carried out as described in Example 5, except that the enzyme used was Lipase from wheat germ (3 g).

The mixture was stirred at 25° C for 30 h (pH = 7.5), obtaining after chromatography 3.4 g (80%) of product.

EXAMPLE 7

The reaction was carried out as described in Example 5, except that the enzyme used was Protease (from Rhizopus Sp., 3 g).

The mixture was stirred at 30° C for 20 h (pH = 7.5), affording, after chromatography, 2.9 g (70%) of product.

EXAMPLE 8

5 g of allyl-(5R,6S)-2--butyryloxymethyl-6-[1-(R)-tetrahydropyranyloxyethyl]-penem-3-carboxylate were added to 300 ml of phosphate buffer 0.05N (pH = 7.0). The mixture was added with 25 mg of Lipase from Chromobacterium viscosum and stirred at 30° C for 4 hours. The pH was kept at 7.0 by addition of 1N NaOH.

At the end of the reaction, the reaction mixture was extracted with $CH_2Cl_2$ (3 x 100 ml), the organic layer was dried over sodium sulfate and evaporated to give allyl-(5R,6S)-2-hydroxy ethyl-6-[1(R)-tetrahydropyranyloxymethyl]-penem-3-carboxylate with a yield of 94%.

H-NMR (300 MHz, $CHCl_3$) - δ (ppm) :

1.3-1.4 (3H, m, $CH_3CH$)
1.45-1.9 (6H, m, $CH_2CH_2CH_2$ of the THP group)
3.42-3.92 (2H, m, $CH_2O$ of THP group)
3.6 (1H,br ,OH)
3.8 (1H, m, H-6)
4.05-4.22 (1H, m, H-8)
4.57-4.82(5H:COO $CH_2$ =; $CH_2OH$; CH of THP group)
5.2-5.45 (2H, m, = $CH_2$)
5.61 (1H, m, H-5)
5.85-6.0 (1H, m, CH=)

EXAMPLE 9

40 g of Amberlite XAD-7 were added to a solution of 100 mg Lipase (from Chromobacterium) in 100 ml 0.01N phosphate buffer (pH = 7.5).

The resin mixture was gently stirred overnight at room temperature.

Then the resin was filtered and washed with 100 ml of the same buffer.

The immobilized enzyme resin was added to a suspension of 20 g of allyl (5R,6S)-2-butyryloxymethyl-6-[1(R)-trimethylsilyloxyethyl]-penem-3-carb xylate in 20 ml hexane and 600 ml 0.01N phosphate buffer (pH = 7.5).

The mixture was stirred at 30° C for 4 hours.

The pH was kept at 7.50 by addition of 1N NaOH. At the end of the reaction, the resin containing the enzyme and the reaction product was separated off by filtration in vacuo through a glass filter and washed with methylene chloride (3 x 300 ml).

The organic extracts were dried over $Na_2SO_4$ and evaporated, affording 15.2g (91%) of product.

The immobilized enzyme resin was washed with phosphate buffer (3 x 200 ml) and used for 6 production cycles without appreciable loss of activity.

## Claims

1. A process for preparing a compound of the formula I :

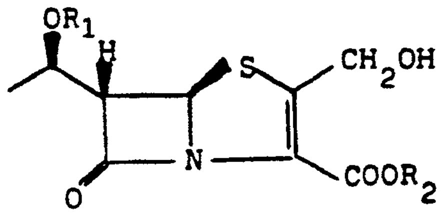

I

wherein $R_1$ represents a hydroxy protecting group and $R_2$ represents a carboxy protecting group, which process comprises hydrolysing a compound of the formula II :

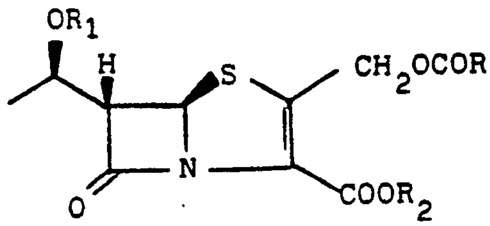

II

wherein $R_1$ and $R_2$ are as defined above and R represents an alkyl, alkenyl or phenylalkyl group having from 1 to 18 carbon atoms, by means of an enzyme capable of selectively hydrolysing the ester group of the 2-substituent thereof.

2. A process according to claim 1, in which $R_1$ represents p-nitrobenzyloxycarbonyl, trimethylsilyl or pyranyl group, $R_2$ represents allyl, benzyl or p-nitrobenzyl group and R represents methyl, ethyl, propyl or butyl group.

3. A process according to claim 1 or 2, in which the enzymatic hydrolysis is carried out by means of a lipase, a protease, pancreatin or steapsin.

4. A process according to any one of the preceding claims, in which the enzymatic hydrolysis is carried out either in the presence of microbial cells which secrete a said enzyme or by isolating a said enzyme and then by using it on the compound of the formula II.

5. A process according to claim 4, in which the microbial cells or the isolated enzyme are either immobilized on an inert substrate or insolubilized by cross-linkage.

6. A process according to claim 5, in which the final compound of the formula I is adsorbed on the inert substrate and then recovered in pure form by simply washing the substrate with a suitable solvent.

7. A process according to any one of the preceding claims, in which the enzymatic hydrolysis is carried out in aqueous solution, the concentration of compound of the formula II being from 1 to 100 g/l, optionally in presence of a small amount of hydrocarbons, buffered at pH from 5 to 9, at the temperature of from 10° to 50°C for a period of from 0.5 to 48 hours.

**Revendications**

1. Procédé de préparation d'un composé de la formule (I) :

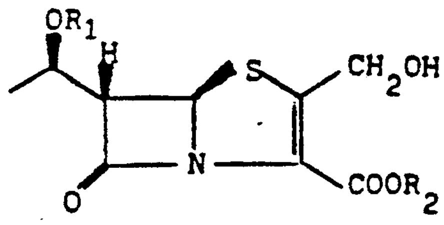

(I)

dans laquelle :

- $R_1$ représente un groupe protecteur d'hydroxy; et
- $R_2$ représente un groupe protecteur de carboxy,

lequel procédé comprend l'hydrolyse d'un composé de la formule (II) :

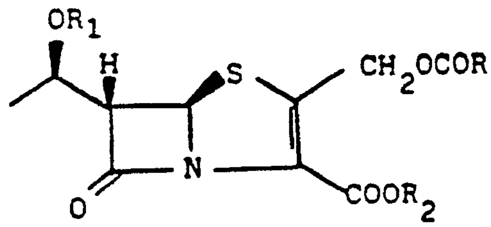

(II)

dans laquelle :

- $R_1$ et $R_2$ sont tels que définis ci-dessus ; et
- R représente un groupe alkyle, alcényle ou phénylalkyle, ayant de 1 à 18 atomes de carbone,

à l'aide d'une enzyme capable d'hydrolyser de manière sélective le groupe ester du substituant en position 2 de ce composé.

**2.** Procédé selon la revendication 1, dans lequel $R_1$ représente un groupe p-nitrobenzyloxycarbonyle, triméthylsilyle ou pyrannyle, $R_2$ représente un groupe allyle, benzyle ou p-nitrobenzyle et R représente un groupe méthyle, éthyle, propyle ou butyle.

**3.** Procédé selon la revendication 1 ou 2, dans lequel l'hydrolyse enzymatique est effectuée à l'aide d'une lipase, une protéase, la pancréatine ou la stéapsine.

**4.** Procédé selon l'une des revendications précédentes, dans lequel l'hydrolyse enzymatique est effectuée soit en présence de cellules microbiennes qui sécrètent ladite enzyme, soit par isolement de ladite enzyme puis par utilisation de celle-ci sur le composé de la formule (II).

**5.** Procédé selon la revendication 4, dans lequel les cellules microbiennes ou l'enzyme isolée sont soit immobilisées sur un substrat inerte, soit insolubilisées par réticulation.

**6.** Procédé selon la revendication 5, dans lequel le composé final de la formule (I) est adsorbé sur le substrat inerte, puis récupéré sous une forme pure par simple lavage du substrat avec un solvant approprié.

**7.** Procédé selon l'une des revendications précédentes, dans lequel l'hydrolyse enzymatique est effectuée en solution aqueuse, la concentration du composé de la formule (II) allant de 1 à 100 q/l, facultativement en présence d'une petite quantité d'hydrocarbures, tamponnée à un pH de 5 à 9, à la température de 10 °C à 50 °C, pendant une période de temps allant de 0,5 à 48 heures.

## Patentansprüche

**1.** Verfahren zur Herstellung einer Verbindung der Formel (I)

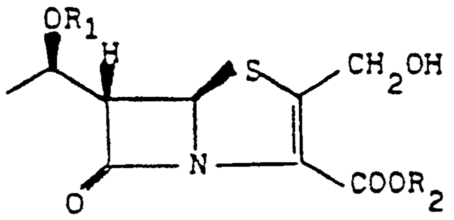

(I)

wobei $R_1$ eine Hydroxy-Schutzgruppe und $R_2$ eine Carboxy-Schutzgruppe darstellen, wobei das Verfahren die Hydrolyse einer Verbindung der Formel (II)

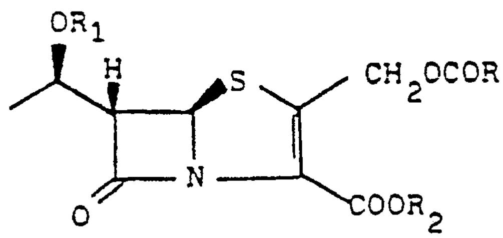

(II)

wobei $R_1$ und $R_2$ wie oben definiert sind und R eine Alkyl-, Alkenyl- oder Phenylalkylgruppe mit 1 bis 18 Kohlenstoffatomen darstellt, mittels eines Enzyms, das in der Lage ist, die Estergruppe des 2-Substituenten davon zu hydrolysieren, umfasst.

2. Verfahren gemäss Anspruch 1, wobei $R_1$ eine p-Nitrobenzyloxycarbonyl-, Trimethylsilyl- oder Pyranylgruppe, $R_2$ eine Allyl-, Benzyl- oder p-Nitrobenzylgruppe und R eine Methyl-, Ethyl-, Propyl- oder Butylgruppe darstellen.

3. Verfahren gemäss Ansprüchen 1 oder 2, wobei die enzymatische Hydrolyse mittels Lipase, Protease, Pancreatin oder Steapsin durchgeführt wird.

4. Verfahren gemäss einem der vorhergehenden Ansprüche, wobei die enzymatische Hydrolyse entweder in Gegenwart mikrobieller Zellen, die das genannte Enzym abscheiden, oder durch Isolierung des genannten Enzyms und anschliessender Anwendung auf die Verbindung der Formel (II) durchgeführt wird.

5. Verfahren gemäss Anspruch 4, wobei die mikrobiellen Zellen oder das isolierte Enzym entweder auf einem inerten Substrat immobilisiert oder durch Vernetzung insolubilisiert werden.

6. Verfahren gemäss Anspruch 5, wobei das Endprodukt der Formel (I) auf dem inerten Substrat adsorbiert and dann durch einfaches Waschen des Substrates mit einem geeigneten Lösungsmittel gewonnen wird.

7. Verfahren gemäss einem der vorhergehenden Ansprüche, wobei die enzymatische Hydrolyse in wässriger Lösung, in der die Konzentration der Verbindung der Formel (II) zwischen 1 und 100 g/l beträgt, gegebenenfalls in Gegenwart einer kleinen Menge an Kohlenstoffen, gepuffert auf einen pH-Wert von 5 bis 9 und bei einer Temperatur von 10 bis 50° C während eines Zeitraums von 0,5 bis 48 Stunden durchgeführt wird.